# EUROPEAN PATENT APPLICATION

(11) **EP 1 724 360 A1**
(43) Date of publication of application: **22.11.2006**
(21) Application number: 05447115.6
(22) Date of filing: 17.05.2005
(51) Int. Cl.: C12Q 1/68

(54) **Identification and/or quantification method of nucleotide sequence(s) elements specific of genetically modified plants on arrays**

(71) Applicant: Eppendorf Array Technologies S.A., 5000 Namur (BE)
(72) Inventor: Remacle, José, 5020 Malonne (BE); Hamels, Sandrine, 1474 Ways (BE)
(74) Representative: Van Malderen, Joëlle

(57) **Abstract**

The present invention is related to an identification and/or quantification method of several organisms among many other ones possibly present in the analyzed sample having homologous sequence(s) by the determination of the genetic map of the organism. The method combines a limited number of amplifications of target sequence(s) using common primer pairs and the recording upon an array for the presence of single signals resulting from the binding between the capture sequence(s) and their corresponding target sequence(s) and correlating the presence of said detected target sequence(s) to the identification of some genetic specific sequence(s) of said (micro)organism(s) referred as genetic elements and from there to the identification of the organism. The method and device according to the invention allow the easy identification/detection of a sequence specific of an organism among other homologous sequence(s) and possibly its quantification.

### Annex to the application documents - subsequently filed sequences listing

SEQUENCE LISTING

<110> Eppendorf Array Technologies SA

<120> Identification and/or quantification method of nucleotide
sequence(s) elements specific of genetically modified plants on
arrays

<130> BP.EATE.007A/EP

<160> 56

<170> PatentIn version 3.3

<210> 1
<211> 21
<212> DNA
<213> Artificial Sequence

<220>
<223> Forward Tnos primer

<400> 1
ttgaatcctg ttgccggtct t 21

<210> 2
<211> 24
<212> DNA
<213> Artificial Sequence

<220>
<223> Reverse Tnos primer

<400> 2
cgctatattt tgttttctat cgcg 24

<210> 3
<211> 22
<212> DNA
<213> Artificial Sequence

<220>
<223> Forward P35S primer

<400> 3
cgtcttcaaa gcaagtggat tg 22

<210> 4
<211> 22
<212> DNA
<213> Artificial Sequence

<220>
<223> Reverse P35S primer

<400> 4
tcttgcgaag gatagtggga tt 22

<210> 5
<211> 19
<212> DNA
<213> Artificial Sequence

<220>
<223> Forward nptII primer

<400> 5
ctcgacgttg tcactgaag 19

<210> 6
<211> 20

<212>  DNA
<213>  Artificial Sequence

<220>
<223>  Reverse nptII primer

<400>  6
gatggatact ttctcggcag                                                    20


<210>  7
<211>  18
<212>  DNA
<213>  Artificial Sequence

<220>
<223>  PCR control forward primer

<400>  7
ccacctgctg accccgtc                                                      18


<210>  8
<211>  19
<212>  DNA
<213>  Artificial Sequence

<220>
<223>  PCR control reverse primer

<400>  8
gggaccctcg cccagaaac                                                     19


<210>  9
<211>  21
<212>  DNA
<213>  Artificial Sequence

<220>
<223>  Forward CaMV primer

<400>  9
gttgttctat tagttgctct t                                                  21


<210>  10
<211>  22
<212>  DNA
<213>  Artificial Sequence

<220>
<223>  Reverse CaMV primer

<400>  10
atggctaatc ttaatcagat cc                                                 22


<210>  11
<211>  21
<212>  DNA
<213>  Artificial Sequence

<220>
<223>  Forward Pnos-nptII primer

<400>  11
cctcggtatc caattagagt c                                                  21


<210>  12
<211>  21
<212>  DNA
<213>  Artificial Sequence

<220>

<223>  Reverse Pnos-nptII primer

<400>  12
ttgtctgttg tgcccagtca t                                                21


<210>  13
<211>  21
<212>  DNA
<213>  Artificial Sequence

<220>
<223>  Forward Pat primer

<400>  13
gaggcgcaag gttttaagtc t                                                21


<210>  14
<211>  20
<212>  DNA
<213>  Artificial Sequence

<220>
<223>  Reverse Pat primer

<400>  14
catcatgcca tccaccatgc                                                  20


<210>  15
<211>  20
<212>  DNA
<213>  Artificial Sequence

<220>
<223>  Forward Cry1Ab primer

<400>  15
cmcwcagaac aacaaygtgc                                                  20


<210>  16
<211>  20
<212>  DNA
<213>  Artificial Sequence

<220>
<223>  Reverse Cry1Ab primer

<400>  16
gwgcwckgat gatgctcacg                                                  20


<210>  17
<211>  19
<212>  DNA
<213>  Artificial Sequence

<220>
<223>  EPSPS primer1

<400>  17
caagtcgmty tccmaccgg                                                   19


<210>  18
<211>  20
<212>  DNA
<213>  Artificial Sequence

<220>
<223>  EPSPS primer2

<400>  18
ccttgcccgt attgatgacg                                                  20

<210>  19
<211>  19
<212>  DNA
<213>  Artificial Sequence

<220>
<223>  EPSPS primer3

<400>  19
gtcaaggacc gcattgcga                                                    19


<210>  20
<211>  26
<212>  DNA
<213>  Artificial Sequence

<220>
<223>  P35S capture nucleotide sequence

<400>  20
gtcatccctt acgtcagtgg agatat                                            26


<210>  21
<211>  26
<212>  DNA
<213>  Artificial Sequence

<220>
<223>  Tnos capture nucleotide sequence

<400>  21
gagatgggtt tttatgatta gagtcc                                            26


<210>  22
<211>  23
<212>  DNA
<213>  Artificial Sequence

<220>
<223>  nptIIA capture nucleotide sequence

<400>  22
gggactggct gctattgggc gaa                                               23


<210>  23
<211>  23
<212>  DNA
<213>  Artificial Sequence

<220>
<223>  gut (PCR control) capture nucleotide sequence

<400>  23
gggactggct gctattgggc gaa                                               23


<210>  24
<211>  25
<212>  DNA
<213>  Artificial Sequence

<220>
<223>  Pat1 capture nucleotide sequence

<400>  24
ctgtgtatcc caaagcctca tgcaa                                             25


<210>  25
<211>  24

<212>  DNA
<213>  Artificial Sequence

<220>
<223>  cry1 capture nucleotide sequence

<400>  25
cagacggtgg ctgaagccct gtcg                                              24


<210>  26
<211>  24
<212>  DNA
<213>  Artificial Sequence

<220>
<223>  cry2 capture nucleotide sequence

<400>  26
gagcctgtgg gaaaaaccct gcct                                              24


<210>  27
<211>  24
<212>  DNA
<213>  Artificial Sequence

<220>
<223>  cry3 capture nucleotide sequence

<400>  27
caacctgtgg gagaatcctt gcct                                              24


<210>  28
<211>  23
<212>  DNA
<213>  Artificial Sequence

<220>
<223>  epsps7 capture nucleotide sequence

<400>  28
ctcctactcg ccgccctgtc cga                                               23


<210>  29
<211>  23
<212>  DNA
<213>  Artificial Sequence

<220>
<223>  epsps8 capture nucleotide sequence

<400>  29
ttcatgttcg gcggtctcgc gag                                               23


<210>  30
<211>  23
<212>  DNA
<213>  Artificial Sequence

<220>
<223>  nptIIh capture nucleotide sequence

<400>  30
ccgcttgggt ggagaggcta ttc                                               23


<210>  31
<211>  25
<212>  DNA
<213>  Artificial Sequence

<220>

<223>  CaMV capture nucleotide sequence

<400>  31
cghtttcatg gatttttggt cactg                                          25


<210>  32
<211>  19
<212>  DNA
<213>  Artificial Sequence

<220>
<223>  Forward rubisco acitvase gene primer Pra1

<400>  32
acaaccagat ggtbaacgc                                                 19


<210>  33
<211>  19
<212>  DNA
<213>  Artificial Sequence

<220>
<223>  Reverse rubisco acitvase gene primer Pra2

<400>  33
gcccagtaya arttctcca                                                 19


<210>  34
<211>  20
<212>  DNA
<213>  Artificial Sequence

<220>
<223>  Forward sucrose synthase gene primer Pss1


<220>
<221>  misc_feature
<222>  (15)..(15)
<223>  n is a, c, g, or t

<400>  34
ggtttggaga rrggntgggg                                                20


<210>  35
<211>  20
<212>  DNA
<213>  Artificial Sequence

<220>
<223>  Reverse sucrose synthase gene primer Pss2

<400>  35
tccaadatgt avacaacctg                                                20


<210>  36
<211>  24
<212>  DNA
<213>  Artificial Sequence

<220>
<223>  Maize TPss8 capture nucleotide sequence

<400>  36
agaggaggtg gatagtctcc tgtg                                           24


<210>  37
<211>  24
<212>  DNA
<213>  Artificial Sequence

<220>
<223>  Soybean TPss9 capture nucleotide sequence

<400>  37
agagaagttg aattgactca agga                                              24


<210>  38
<211>  24
<212>  DNA
<213>  Artificial Sequence

<220>
<223>  Rice TPss7 capture nucleotide sequence

<400>  38
gaagcaagtg gatggtgtca agca                                              24


<210>  39
<211>  24
<212>  DNA
<213>  Artificial Sequence

<220>
<223>  Rapeseed OTRcol2 capture nucleotide sequence

<400>  39
tgatgggaac acgtgcgttt tctt                                              24


<210>  40
<211>  24
<212>  DNA
<213>  Artificial Sequence

<220>
<223>  Tomato PTRtom4 capture nucleotide sequence

<400>  40
gtaccctggc gttctcttgc ttgt                                              24


<210>  41
<211>  24
<212>  DNA
<213>  Artificial Sequence

<220>
<223>  Sugar beet PTRbett2 capture nucleotide sequence

<400>  41
tgatgggtac acgagcattg tcct                                              24


<210>  42
<211>  22
<212>  DNA
<213>  Artificial Sequence

<220>
<223>  Forward soybean lectin primer

<400>  42
cattacctat gatgcctcca cc                                                22


<210>  43
<211>  19
<212>  DNA
<213>  Artificial Sequence

<220>
<223>  Reverse soybean lectin primer

<400>  43
aagcacgtca tgcgattcc                                                    19


<210>  44
<211>  20
<212>  DNA
<213>  Artificial Sequence

<220>
<223>  Forward rapeseed cruciferin primer

<400>  44
agagacgaag gaagcgaagg                                                   20


<210>  45
<211>  20
<212>  DNA
<213>  Artificial Sequence

<220>
<223>  Reverse rapeseed cruciferin primer

<400>  45
tgacccatct aatgctgacg                                                   20


<210>  46
<211>  19
<212>  DNA
<213>  Artificial Sequence

<220>
<223>  Forward maize invertase primer

<400>  46
gcgctctgta caagcgtgc                                                    19


<210>  47
<211>  21
<212>  DNA
<213>  Artificial Sequence

<220>
<223>  Reverse maize invertase primer

<400>  47
gcaaagtgtt gtgcttggac c                                                 21


<210>  48
<211>  21
<212>  DNA
<213>  Artificial Sequence

<220>
<223>  Forward tomato rDNA primer

<400>  48
gtttcaaaag taacacggca a                                                 21


<210>  49
<211>  22
<212>  DNA
<213>  Artificial Sequence

<220>
<223>  Reverse tomato rDNA primer

<400>  49
ggcttgataa atgaactcaa ct                                                22

<210>  50
<211>  20
<212>  DNA
<213>  Artificial Sequence

<220>
<223>  Forward plant universal Rbcl primer

<400>  50
agycttgatc gttacaaagg                                                    20


<210>  51
<211>  20
<212>  DNA
<213>  Artificial Sequence

<220>
<223>  Reverse plant universal Rbcl primer

<400>  51
aggtctaadg grtaagctac                                                    20


<210>  52
<211>  23
<212>  DNA
<213>  Artificial Sequence

<220>
<223>  Soybean lectin capture nucleotide sequence

<400>  52
ctgccacggg actcgacata cct                                                23


<210>  53
<211>  25
<212>  DNA
<213>  Artificial Sequence

<220>
<223>  Rapeseed cruciferin capture nucleotide sequence

<400>  53
ttcagagtgc tgatgtaacc gagct                                              25


<210>  54
<211>  24
<212>  DNA
<213>  Artificial Sequence

<220>
<223>  Maize invertase capture nucleotide sequence

<400>  54
ttagacggga aaacgagagg aagc                                               24


<210>  55
<211>  23
<212>  DNA
<213>  Artificial Sequence

<220>
<223>  Tomato rDNA capture nucleotide sequence

<400>  55
ctcgcaatgg ggctcagaac gca                                                23


<210>  56
<211>  55
<212>  DNA
<213>  Artificial Sequence

<220>
<223>  Universal plant capture nucleotide sequence

<400>  56
ataagcaata tattgatttt cttctccagc aacgggctcg atgtggtagc atcgc          55

## Description

### Field of the invention

The present invention is in the field of diagnosis and is related to a method and means for the identification and/or the quantification of nucleotide sequence(s) elements specific of genetically modified plant(s), possibly present in a biological sample, and wherein said nucleotide sequence(s) elements could be homologous, in different genetically modified plants.

The invention is especially suited for the identification and/or quantification of organisms of the same genus or the same family and for the detection and/or quantification of organisms being genetically modified.

### Background of the invention

The development of the biochips technology allows the detection of multiple nucleotide sequence(s) simultaneously in a given assay and thus allows the identification of the corresponding organism or part of the organism. However detections have to be worked out in conjunction with the amplification by PCR and there is not much advantages to perform one PCR for each organism and to use the microarray for detection. Thus, a strategy for detection of a large number of organisms has to be worked out in order to lower the number of PCR reactions while keeping the identification of multiple organism.

### State of the art

The Company Affymetrix Inc. has developed a method for direct synthesis of oligonucleotides upon a solid support, at specific locations by using masks at each step of the processing. Said method comprises the addition of a new nucleotide on a growing oligonucleotide in order to obtain a desired sequence at a desired location. This method is derived from the photolithographic technology and is coupled with the use of photoprotective groups, which are released before a new nucleotide is added (EP-A1-0476014, US-A-5,445,934, US-A-5,143,854 and US-5,510,270). However, only small oligonucleotides are present on the surface, and said method finds applications mainly for sequencing or identifying a pattern of positive spots corresponding to each specific oligonucleotide bound on the array. The characterization of a target sequence is obtained by comparison of such pattern with a reference. Said technique was applied to the identification of Mycobacterium tuberculosis rpoB gene (WO97/29212 and WO98/28444), wherein the capture nucleotide sequence comprises less than 30 nucleotides and from the analysis of two different sequence(s) that may differ by a single nucleotide (the identification of SNPs or genotyping). Small capture nucleotide sequence(s) (having a length comprised between 10 and 20 nucleotides) are preferred since the discrimination between two oligonucleotides differing in one base is higher, when their length is smaller.

The lack of sensitivity of the method is illustrated by the fact that it cannot detect directly amplicons resulting from genetic amplification (PCR). A double amplification with primer(s) bearing a T3 or T7 sequence(s) and then a retrotranscription with a RNA polymerase. These RNA are cut into pieces of about 40 bases before being detected on an array (example 1 of WO 97/29212). However, long DNA or RNA fragments hybridize very slowly on capture probes present on a surface. Said methods are therefore not suited for the detection of homologous sequence(s) since the homology varies along the sequence(s) and so part of the pieces could hybridize on the same capture probes. Therefore, a software for the interpretation of the results should be incorporated in the method for allowing interpretation of the obtained data.

However, for gene expression array which is based on the cDNA copy of mRNA the same problem is encountered when using small capture probe arrays: the rate of hybridisation is low. Therefore, the fragments are cut into smaller species and the method requires the use of several capture nucleotide sequence(s) in order to obtain a pattern of signals which attest the presence of a given gene (WO97/10364 and WO97/27317). Said cutting also decreases the number of labelled nucleotides, and thus reduces the obtained signal. In this case, the use of long capture nucleotide sequence(s) give a much better sensitivity to the detection. In the many gene expression applications, the use of long capture probes is not a problem, when cDNA to be detected originates from genes having different sequence(s), since there is no cross-reactions between them. Long capture nucleotide sequence(s) give the required sensitivity, however, they will hybridize to other homologous sequence(s).

Arrays are well suited for multiparametric detections but since the materials in biological samples are in very low amount, a strategy has to be worked out in order for the combination between the PCR and the array to be efficient. Making a PCR for each possible organism possibly present in the analyzed sample and then detected and/or identified the amplified sequence(s) individually is not very efficient and is part of the art. When multiple organisms are possibly present in a sample, the number of primers to be used has to be limited since it will lead to either a large number of PCR if realized in different tubes or to non specific amplificaiton when mulitplex PCR are performed; Therefore, there is a need to simplify the PCR and to develop a strategy of combination between the PCR and the array design.

Such situation is present in the detection and/or identification of Genetically Modified (GM) Plants or Organisms (GMO) in biological samples. A GMO is defined by a unique transformation event that usually means insertion of a heterologous gene construct into the recipient organism. The integrator gene construct is composed of several elements, usually at least a gene of interest, a promoter functioning as start signal, and a terminator functioning as a stop signal for regulation of gene expression. In addition, the construct may be flanked by DNA sequences from the cloning vector. The majority of genetically modified plants have been transformed with constructs containing the Cauliflower Mosaic Virus (CaMV) 35S promoter (P-35S) and/or the CaMV 35S terminator (T-35S) or the *Agrobacterium tumefaciens* nopaline synthase terminator (T-Nos). The most commonly used cloning vectors, are pBR322 containing a gene coding for resistance to ampicillin (bla) antibiotics, or vectors containing a gene coding for resistance to neomycin/kanamycin (*nptII*) antibiotics.

Several countries have mandatory labelling regulations for GMO and for GM-derived food. Particularly, European regulations establish the compulsory labelling of food and feed consisting of or containing genetically modified organisms (GMO) in a proportion higher than 0.9 percent of the food ingredients (Regulation EC no. 1829/2003). This 0.9% limit for labelling lead to a detection limit to be 0.1% or even lower in order to take into account the variability of the assays. Such limit corresponds to a few hundreds or even lower of copy of genome in the sample.

In order to comply with the requests of those regulations and to guarantee to the consumers a freedom of choice by accurate information, several GMO analytical methods have been already described and are currently used by European enforcement laboratories. Because the products that laboratories receive for analysis are often processed and refined, the quality and quantity of target analyte frequently challenges the sensitivity of any detection method. Among the currently available methods, PCR methods followed by electrophoresis are generally accepted as the most sensitive and reliable methods for the detection of GM-derived material in routine applications.

GMO testing may be limited to a simple screening PCR. However, specific identification of the GMO from which DNA is derived, or reliable quantification of the GMO-derived DNA rapidly increases the costs, in particular as the number of GMO to detect is continually increasing.

Because of the sensitivity to be reached, genetic material from the sample is first amplified by PCR before detection. PCR-based GMO tests can be grouped into at least four categories corresponding to their level of specificity. Each category corresponds to the composition of the DNA fragment that is amplified in the PCR: screening targets, gene specific targets, construct specific and event specific targets.

The first category of PCR methods, targeting the P-35S, T-35S, T-Nos, bla or nptII genetic elements, have wide applications for screening for genetically modified material (Matsuoka et al., 2002, J. Agric. Food Chem. 93: 35-38). However, these screening methods cannot be used to identify the GMO, since the presence of one of the screening targets does not necessary imply the presence of GMO-derived DNA. The source of P-35Sor T-35S could be naturally occurring CaMV (Wolf et al. 2000, Eur. Food Res Technol. 210:367-372).

The second category of PCR methods, targeting a gene of interest (e.g. CryIA(b) gene), are more specific than the screening methods (Vaitilingom et al. 1999, J. Agric. Food Chem. 47: 5261-5266). The choice of available genes is greater than the choice of available promoter and terminators. Normally a positive signal for the amplification of a specific gene implies that GM-derived DNA is present, and in many cases it is possible to identify from which GMO the DNA is derived.

The third category of PCR methods targets junction between adjacent elements of the gene construct (specific construct), for example between the promoter and the gene of interest (e.g. Mon810 maize: P-35S-hsp70 intron I) (Zimmerman et al. 1998, Lebensm-Wiss u Technol. 31: 664-667). With these methods a positive signal will only appear in the presence of GM-derived material. However, the full gene construct may have been transformed into more than one GMO, or may be used in future transformation.
The only unique signature of a transformation event, within the limitations of present day technology, is the junction at the integration locus between the recipient genome and the inserted DNA. This junction is the target of the fourth category of PCR methods. Unfortunately, even the event-specific methods have their limitations. When two GMOs are crossed (e.g. two different GM maize such as T25 and Mon810), the resulting hybrid offspring may contain the genetic modifications including signatures of both events and will be indistinguishable from its two parents in a PCR test. One important limitation of this detection is the requirement of one specific primer pair for each GMO to identify.

Some of these assays are based on conventional PCR and allow a qualitative approach of the GMO in a given sample. Other such as the real-time PCR (Hernández et al., 2003, Transgenic Research 12: 179-189) allow the quantification of GMO in a sample.

The major limitations for PCR-based detection of DNA derived from GMOs are access to information about applicable PCR primers and access to DNA suitable for reliable analysis. Although, several PCR primer pairs for GMO analysis have been developed and published, many of these primers have a limited range of application (e.g. primers suitable only for screening or for identification of one GMO).

Processing food such as grinding, heating, acid treatment and other processing rapidly degrades DNA in small fragments of about 200-400 bp, and refining can lead to efficient removal of DNA. Therefore, many products contain little GMO-derived DNA, and this DNA is often fragmented. As a consequence, to be able to detect GMO in processed food, primer pairs should target small fragments (100-200 bp).

There is a need to broader the number of GMOs that could be detected in a single assay. There is also a need for a method that would be easily upgraded for inclusion of new GMO. A multiplex PCR would theoretically allow the simultaneously detection of several GMO. However, such assay is not easy to implement because several small amplified products are not easily discriminated by electrophoresis. Also, a multiplex PCR usually presents a lower sensitivity as compared to single amplification which is an important drawback for GMO detection.

In an attempt to perform a multiparametric assay, the US-20030198943A1 described a method for the identification of PCR-amplified GMO on biochip. The PCR is based on the use of a consensus primer pair (P-35S/T-35S) which delimits very long DNA sequence including several genetic elements (e.g. CTP1, CTP2, CTP4, CP4EPSPS, EPSPS, CryIA(b), hsp70 intron, Pat) found in different GMOs.

A limitation of this method is the small number of GMOs being identified by the method and the impossibility to get an identification since many GMO contain the same element.

### Aims of the invention

The present invention aims to provide a new method (and device) to improve microarrays or biochips technology for the easy identification (detection and/or quantification) of a large number of organisms or portions of organisms having parts of their nucleotide sequence(s) being homologous.

A further aim of the invention is to provide such method (and device) based upon a simplified technology requiring the use of a limited number of primers in an amplification step with the identification (detection and/or quantification) of the specific amplified target sequence(s) by detection and/or recording of single spot signals upon said microarray, said signals resulting from the specific binding of the target sequence(s) with their corresponding capture sequence.

A further aim of the invention is to identify organisms having some parts of their sequence being identical or highly homologous in particular the detection of Genetically Modified Plants (GM plants) or Organisms (GMO).

A last aim of the present invention is to propose such method which simplifies the origin identification of genetic material present in a biological sample, especially from which genetically modified plant the biological sample is obtained.

### Definitions

The terms "nucleic acid, oligonucleotide, array, probe, target nucleic acid, bind substantially, hybridising specifically to, background, quantifying" are the ones described in the international patent application WO97/27317 incorporated herein by reference.

The terms "nucleotide triphosphate, nucleotide, primer sequence" are those described in the document WO00/72018 and PCT/BE00/00123 incorporated herein by references.

The terms "homologous genetic sequence(s)" or "homologous nucleotide sequence(s) elements" mean amino acid or nucleotide sequence(s) having a percentage of amino acids or nucleotides identical at corresponding positions which is higher than in purely random alignments. They are considered as homologous when they show a minimum of homology (or sequence identity) defined as the percentage of identical nucleotides or amino acids found at each position compared to a total of nucleotides or amino acids, after the sequence(s) have been optimally aligned taking into account additions or deletions (like gaps) in one of the two sequence(s) to be compared. Genes coding for a given protein, but present in genetically different sources like different organisms, are usually homologous. Also in a given organism, genes coding for proteins or enzymes of the same family (Interleukins, cytochrome b, p. 450) are usually homologous. The degree of homology (or sequence identity) can vary a lot as homologous sequence(s) may be homologous only in one part, a few parts or portions or all along their sequence(s). The homologous parts or portions of the sequence (s) that are identical in the sequence (s) are perfectly homologous and are also said conserved or identical. Genetic construction as for cell transfection may contain identical or homologous parts, such as sequence(s) portions defined hereafter as nucleotide sequence elements.

These nucleotide sequence(s) elements include regulatory sequence(s), such as terminators, promoters or a gene of interest incorporated into the genome of the plant (antibiotic markers) or sequence(s) involved into production of molecules of interest or parts (portions) of these sequences. These nucleotide sequence elements could be also repeated sequence(s) and/or genes coding for specific enzymatic activities or parts (portions) of these sequences. Protein domains which present a conserved three dimensional structure are usually coded by homologous sequence(s) and even often by a unique exon.

The sequence(s) showing a high degree of invariance in their sequence(s) are said to be highly conserved and they present a high degree of homology. A "genetic map" is the organisation of these various nucleotide sequence elements in the genome of the organism, preferably in the genome of the plant.

A "biological sample" means any medium that can comprise different and multiple nucleotide sequence(s) elements specific of a genetically modified plant, or having a genetically modified plant origin.

For instance, such biological sample could be a food, a food ingredient or a food additive.

The biological sample could be also a solid or a liquid extract which could be contaminated by genetically modified plants. The biological sample could be submitted to an extraction step of these nucleotide sequence(s) elements specific of a type of genetically modified plant.

The terms "genetically modified plants" correspond to all types of plants which have been submitted to some genetic modifications, especially an insertion of a cassette sequence comprising exogenous elements to the plant (promoters and terminators sequences flanking a gene of interest and/or a genetic marker).

### Summary of the invention

The invention related to an identification and/or quantification method of different and multiple nucleotide sequence elements specific of a genetically modified plant in a biological sample, which comprises the steps of :
a. possibly extracting the nucleotide sequence elements from the biological sample;
b. amplifying or copying a nucleotide sequence element into target nucleotide sequences, using primer pairs which are able to amplify the said nucleotide sequence element being homologous with nucleotide sequence elements present in at least two different genetically modified plants;
c. possibly, labelling the obtained target nucleotide sequences;
d. putting into contact the obtained target nucleotide sequences with single stranded capture nucleotide sequences bound by a covalent link to an insoluble solid support at a specific location of a solid support surface;
e. detecting the binding of said target nucleotide sequences, by detecting a signal resulting from hybridization by complementary base pairing of the said target nucleotide sequence and its corresponding capture nucleotide sequence at the specific location, wherein the capture nucleotide sequence(s) are bound to the insoluble solid support at a specific location according to an array, having a density of at least 4 different bound single stranded capture nucleotide sequence(s)/cm² of solid support surface, wherein the capture nucleotide sequence comprises a sequence having between 10 and 200 bases, preferably between 10 and 49 bases which allows a specific hybridization with the target nucleotide sequence to be detected and/or quantified;
f. repeating the steps b) to e) for a second, third, fourth or more different nucleotide sequence elements specific of the said genetically modified plant, and
g. identifying the genetically modified plant by the presence or absence of these different and multiple nucleotide sequence(s) elements, in the biological sample.

The inventors have discovered that for some applications it is possible to drastically simplify the identification of one or several organisms among many other ones possibly present in the analyzed sample having homologous sequence(s) by the determination of the genetic map of the organism using a strategy of combined particular amplifications together with microarray detection. The method combines a limited number of amplification steps using consensus primer pairs and the detection, the quantification and/or the recording upon an array of a single signal corresponding to the presence of a target nucleotide sequence (the binding between a capture nucleotide sequence and its corresponding target nucleotide sequence) and correlating the presence of the detected target nucleotide sequence(s) to the identification (presence or absence) of these nucleotide sequence(s) elements specific of the (micro)organism(s) in the biological sample submitted to the detection and/or quantification.

The method and device according to the invention allow the easy identification/detection of a nucleotide sequence(s) element specific of an organism among other homologous sequence(s) elements, and possibly its quantification (characterisation of the number of copies or presence of these organisms in the biological sample).

The identification is obtained after a binding on specific capture nucleotide sequence(s) present on a support or a substrate, preferably in the form of an array. The identification of the target sequences is obtained directly after washing of possible contaminants (unbound sequence(s)), by detecting and possibly recording for one target sequence, a single spot signal at one specific location (wherein the corresponding capture nucleotide sequence was previously bound), but wherein the target sequence identification is not the result of analysis of a complex pattern of spots upon the microarray and wherein no fragmentation of the amplified target sequences is required.

Single and even double stranded target sequence(s) can be advantageously discriminated from other homologous ones upon an array with high sensitivity by using bound capture nucleotide sequence(s) composed of at least two parts, one being a spacer bound by a single and advantageously predetermined (defined) link to the solid support surface; preferably a non porous support and the other part being a specific nucleotide sequence able to hybridise specifically by complementary base pairing with the amplified nucleotide target sequence.

Furthermore, this detection is greatly increased, if high concentrations of capture nucleotide sequence(s) are bound to the surface of the solid support.

The present invention is related to the identification of nucleotide sequence(s) elements specific from a biological organism or portion thereof (possibly present in a biological sample) from at least 4 other homologous (or identical) nucleotide sequences element(s) obtained from other different organisms or varieties, said other organisms could be present in the same biological sample and have homologous or identical nucleotide sequences element(s) with the elements to be detected and/or quantified in the sample.

Said identification (presence or absence of these elements in the sample) is obtained firstly by genetic amplifications of the nucleotide sequences element(s) by several consensus primer pairs, followed (after washing) by discrimination between the possible different amplified target sequences obtained from these amplification steps. This discrimination is advantageously obtained by hybridization upon an array of capture nucleotide sequence(s) bound to the solid support surface at given (specific and pre-determinate) locations.

Through the detection and possibly the recording of signal(s) resulting from the specific binding of these target sequences upon their corresponding capture nucleotide sequences at the expected locations (a single location signal being specific for the binding of a specific target sequence), the presence and/or the absence of the different target nucleotide sequences allows to determine the presence and/or the absence of different specific nucleotide sequence element(s) is the sample.

The method according to the invention further comprises the step of correlating the presence and/or the absence of the nucleotide sequence element(s) in the sample to the presence and/or the identification of:
- specific organism(s),
- genetically Modified Organism (GMO), especially genetically modified plants,
- genetic characteristics (mutation, deletion, insertion) in a sequence,
- diagnostic predisposition or evolution (monitoring) of genetic diseases, including cancer of a patient (including the human) from which the biological sample has been obtained.

The method is particularly suitable for the detection and screening of GMO's or other organisms or parts of them in the biological sample having some parts (elements) of their genomic sequence(s) identical or highly homologous (preferably having more than 80% and even more than 90% homology), and some parts different and for which a large number of such organisms are possibly present in a sample.

The present invention allows in one single assay the identification of the nucleotide sequence element(s) (after their amplification into target nucleotide sequences) on an array for the presence and absence following the detection. Said detection is preferably compared to a determination of the genetic maps of these organisms.

Indeed, the presence or absence of some or all of the detected elements in the different genetic maps of the organisms allows the identification of the specific organism which is present in the biological sample. The presence or absence of these elements could be also correlated with a data bank or table, comprising all the genetic maps of the searched elements for the proper identification of each organism presented in the databank.

In a particular embodiment, the method also provides means for the detection and/or identification of an organism being part of a family, having specific genetic elements but being classified as not being part of a data bank having the genetic map of each of the organisms to be detected.

In a preferred embodiment, the method provides also means in the form of a computer program for the analysis of the experimental results obtained on the detection and/or identification of different elements and the identification of an organism. The program also provides preferably a data bank with the composition of the elements of the possibly detectable organisms.

In a preferred embodiment the elements of the different genetic map of the organisms are arranged into a table and the results of the assays are compared one by one with the table data.

In another embodiment the present invention is suitable for the determination of an organism having one or several searched elements, but not for all the elements characteristics of the searched organisms. The organisms will then be cited as unknown organisms.

The invention preferably requires the table including the present elements and the method for sorting out the identification of the organism from the experimental data.

In a particular embodiment, the sequences from different organisms are amplified in total or in part with the same primers and the obtained target sequences are detected on a common capture sequence.

In another embodiment, the target sequences originated from different organisms are amplified in total or in part with the same primers, but are detected on specific capture sequences.

In still another preferred embodiment, the arrays contain both capture sequences specific for the same (amplified) target nucleotide sequences of different organisms and capture sequences common for the same (amplified) target sequences obtained from different organisms.

In a particular embodiment the invention is performed on GMO plants having inserted exogenous nucleotide sequence elements, as provided in table I and II. The elements of the GMO's are detected as provided by the method of the present invention. In a particular application, 6 elements (or parts of them) being P35s, T-nos, nptII, pat, Cry1Ab, EPSPS are amplified in 3 different tubes with 6 primers pairs as provided in example 2. The means advantageously includes appropriate controls for the detection of possible presence of CaMV and for the identification of the plant species.

In a particular embodiment, the present invention provides a method for the identification of a number of organisms being larger than the number of (amplified) target nucleotide target sequence(s), the number of organisms to be able to be detected being the number of amplifications plus at least 2 and better plus 5 and even better than 10.

The present method applies for 9 GMO detection and/or identification (of table 1) and for the detection and/or identification of 21 GMO (of table 2).

Example for the identification of the GMO organisms by comparing two by two the genetic elements to be detected in the example 3 is given in table 3. In this example and given the particular elements detected on the microarray, the genetic mapping is specific for each GMO except for 3 GMO organisms which showed identical mapping with another organism. Results present in table 4 shows that the GMO can be quantified and that the level of detection is lower than 0.1 % of GMO present in a sample. The method is particularly suitable for multiple and complex analysis of organisms present at low concentrations in the biological sample.

According to the invention, the preferred method for genetic amplification is the PCR using two antiparallel primers being consensus for a particular sequence to be amplified.

The organisms to be detected and/or quantified are possibly present in any biological material including genetic material obtained (virus, fungi, bacteria, plant or animal cell, including the human). The biological sample can be also any culture medium wherein microorganisms, xenobiotics or pollutants are present, as well as such extract obtained from a plant or an animal (including a human) organ, tissue, cell or biological fluid (blood, serum, urine, etc).

The kit or device according to the invention may also incorporate various media or devices for performing the method according to the invention. Said kit (or device) can also be included in an automatic apparatus such as a high throughput screening apparatus for the detection and/or the quantification of multiple nucleotide sequence(s) present in a biological sample to be analysed. Said kit or apparatus can be adapted for performing all the steps or only several specific steps of the method according to the invention.

The diagnostic and/or quantification kit of the invention may comprises means and media for performing the method of the invention, preferably an insoluble solid support upon which single stranded capture nucleotide sequences are bound, said single stranded capture nucleotide sequences containing sequences of between about 10 and about 200 bases specific for at least three of P35s, T-nos, nptII, pat, Cry1Ab and EPSPS nucleotide sequences to be detected and/or quantified and having a total length comprised between about 30 and about 600 bases, said single stranded capture nucleotide sequence(s) being disposed upon the surface of the solid support according to an array with a density of at least 4 single stranded capture nucleotide sequence(s)/cm² of the solid support surface.

The method according to the invention can be performed by using a specific identification (diagnostic and/or quantification) kit or device comprising at least an insoluble solid support upon which are bound some single stranded capture nucleotide sequence(s) (preferably bound to the surface of the solid support by a direct covalent link or by the intermediate of a spacer) according to an array with a density of at least 4, preferably at least 10, 16, 20, 50, 100, 1000, 4000, 10 000 or more, different capture nucleotide sequence(s)/cm² insoluble solid support surface, said capture nucleotide sequence(s) having advantageously a length comprised between about 30 and about 600 bases (including the spacer) and containing a sequence of about 10 to about 60 bases, said sequence being specific for the target (which means that said bases of said sequence are able to form a binding with their complementary bases upon the sequence of the target by complementary hybridisation). Preferably, said hybridisation is obtained under stringent conditions (under conditions well-known to the person skilled in the art).

In the method, ((kit (device) or apparatus) according to the invention, the portion(s) (or part(ies)) of the capture nucleotide sequence(s) complementary to the target is comprised between about 10 and about 60 bases, preferably between about 15 and about 40 bases and more preferably between about 20 and about 30 bases. These bases are preferably assigned as a continuous sequence located at or near the extremity of the capture nucleotide sequence. This sequence is considered as the specific sequence for the detection. In a preferred form of the invention, the sequence located between the specific capture nucleotide sequence and the support is a non specific sequence.

In the method and (kit or device) according to the invention, the capture nucleotide sequence is a sequence having between 16 and 600 bases, preferably between 30 and 300 bases, more preferably between 40 and 150 bases and the spacer is a chemical chain of at least 6.8 nm long (of at least 4 carbon chains), a nucleotide sequence of more than 20 bases and preferably between 50 and 150 base long or is nucleotide derivative such as PMA.

In the preferred embodiment of the invention, the capture nucleotide sequence(s) are chemically synthesised as single stranded. They are polunucleotides either oligonucleotides shorter than 100 bases or longer than 100 bases single stranded are (easily performed on programmed automatic synthesiser). Such sequence(s) can bear a functionalised group such amine of sulfide for covalent attachment upon the support, at high concentrations.

Long capture nucleotide sequence(s) typically higher than 100 or even higher than 150 nucleotides are preferably synthesised by PCR amplification (of a sequence incorporated into a plasmid containing the specific part of the capture nucleotide sequence and the non specific part (spacer)).

The method, (kit (device) or apparatus) according to the invention are suitable for the detection and/or the quantification of nucleotide sequences which are made of DNA or RNA, including sequence(s) which are partially or totally homologous upon their total length.

In the method, (kit (device) or apparatus) according to the invention, the capture nucleotide sequence(s) are advantageously covalently bound (or fixed) upon the insoluble solid support, preferably by one of their extremities as described hereafter.

The method according to the invention gives significant results which allows identification (detection and quantification) with amplicons in solutions at concentration of lower than about 10 nM, of lower than about 1 nM, preferably of lower than about 0.1 nM and more preferably of lower than about 0.01 nM (= 1 fmole/100 µl).

Another important aspect of this invention is to use very concentrate capture nucleotide sequence(s) on the surface. Arrays are produced by a robot with spotting of very low volume of solution being on the order of 0.1 to 1 nl for spots having a surface of around 0.2 to 0.4 nm in diameter. If too low, the yield of the binding is quickly lower and is undetectable. Concentrations of capture nucleotide sequence(s) between about 600 and about 3,000 nM in the spotting solutions are preferred. However, concentrations as low as about 100 nM still give positive results in favourable cases (when the yield of covalent fixation is high or when the target to be detected is single stranded and present in high concentrations). Such low spotting concentrations would give density of capture nucleotide sequence as low as 20 fmoles per cm². On the other side, higher density was only limited in the assays by the concentrations of the capture solutions, but concentrations still higher than 3,000 nM give good results.

The use of these very high concentrations and long probes are two unexpected characteristic features of the invention. The theory of DNA hybridisation proposed that the rate of hybridisation between two DNA complementary sequence(s) in solution is proportional to the square root of the DNA length, the smaller one being the limited factor (Wetmur, J.G. and Davidson, N. 1968, J. Mol. Biol. 3, 584). In order to obtain the required specificity, the specific sequence(s) of the capture nucleotide sequence(s) had to be small compared to the target. Moreover, the target nucleotide sequence(s) were obtained after PCR amplification and were double stranded so that they reassociate in solution much faster than to hybridise on small sequence(s) fixed on a solid support where diffusion is low thus reducing even more the rate of reaction. It was unexpected to observe a so large increase in the yield of hybridisation with the same short specific sequence.

The amount of target nucleotide sequence(s) which "bind" on the spots is very small compared to the amount of capture nucleotide sequence(s) present. So there is a large excess of capture nucleotide sequence and there was no reason to obtain the binding if even more capture nucleotide sequence(s).

One may perform the detection on the full length sequence after amplification or copy and when labelling is performed by incorporation of labelled nucleotides, more markers are present on the hybridised target making the assay sensitive.

The method, (kit and apparatus) according to the invention may comprise the use of other bound capture nucleotide sequence(s), which may have the same characteristics as the previous ones and may be used to identifying a target sequence from another group of homologous sequence(s) (preferably amplified by common primer(s)).

Detection of other sequence(s) can be advantageously performed on the same array (i.e. by allowing an hybridisation with a standard nucleotide sequence used for the quantification, with consensus capture nucleotide sequence(s) for the same targets. Said other capture nucleotide sequence(s) have (possibly) a specific sequence longer than 10 to 60 bases and a total length as high as 600 bases and are also bound upon the insoluble solid support (preferably in the array made with the other bound capture nucleotide sequence(s) related to the invention). A long capture nucleotide sequence may also be present on the array as consensus capture nucleotide sequence for hybridisation with all sequence(s) of a given genetic element, thus giving information on the presence or not of an organism of a family, in the biological sample.

The solid support according to the invention is made with materials selected from the group consisting of gel layers, glasses, electronic devices, silicon or plastic support, polymers, compact discs, metallic supports or a mixture thereof (see EP 0 535 242, US 5,736,257, WO99/35499, US 5,552,270, etc). Advantageously, said solid support is a single glass slide which may comprise additional means (barcodes, markers, etc.) or media for improving the method according to the invention.

The amplification step used in the method according to the invention is advantageously obtained by well known amplification protocols, preferably selected from the group consisting of PCR, RT-PCR, LCR, CPT, NASBA, ICR or Avalanche DNA techniques.

Advantageously, the target nucleotide sequence to be identified is labelled previously to its hybridisation with the single stranded capture nucleotide sequence(s). Said labelling (with known techniques from the person skilled in the art) is preferably also obtained upon the amplified target nucleotide sequence previously to the denaturation (if the method includes an amplification step).

Advantageously, the length of the target nucleotide sequence is selected as being of a limited length preferably between 50 and 800 bases, preferably between 100 and 400 bases and more preferably between 100 and 200 bases. This preferred requirement depends on the possibility to find consensus primers to amplify the required sequence(s) possibly present in the sample. Too long nucleotide sequence target may reallocate faster and adopt secondary structures which can inhibit the fixation on the capture nucleotide sequence(s).

Detection of genes is also a preferred application of this invention. The detection of homologous genes is obtained by first reverse transcription of the mRNA and then amplification by consensus primers as described in this invention.

According to a further aspect of the present invention, the method, (kit (device) or apparatus) according to the invention is advantageously used for the identification of different *GMO's* present together or separately in the biological sample, said identification being obtained by detecting the nucleotide sequence element(s) incorporated into the plants or animals or bacteria or yeast by genetic modification of their genome.

Preferably, the primer(s) and the specific portions of said GMO sequence(s) to be amplified and detected are given in the example 2 and 3. These primers amplified part or total sequence of at least 3 of the following nucleotide sequence element(s) when present in the sample being P35s, T-nos, nptII, pat, Cry1Ab and EPSPS, then detected on the array.

According to a further aspect of the present invention, the method, (kit (device) or apparatus) according to the invention is advantageously used for the identification of at least 4 of the GMOs plants among BT11, BT176, Ga21, Mon 810, RRS, T25, T45, Topas19/2, Starlink, NK603, GT73, Liberator L62, Falcon GS 40/90, MS1-RF1, MS1-RF2, MS8-RF3, 1445, 531, Mon863, Mon810xMON863, TC1507 and Maisgard/RR.

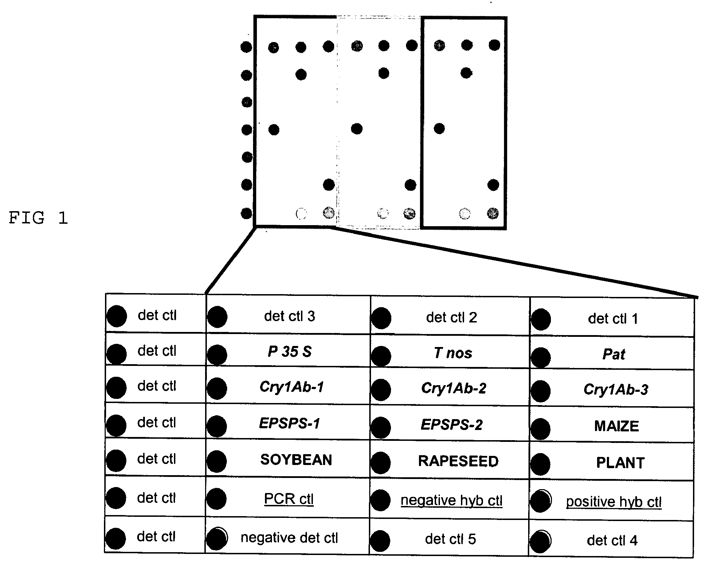

The detection of 9 plant GMO according to the invention is presented in table 1 and in example 2 and 3. The array for performing the detection and identification is presented in figure 1. Also the detection of 21 plant GMO is presented in table 2 and the data bank for individual identification of the GMO is presented in table 3. A list of GMO is provided in data base such are the BATS (http://www.gmo-watch.org/gmo-watch/GVO-report140703.pdf) and AgBios (http://www.agbios.com/dbase.php).

Data bank also provides a list of the nucleotide sequence elements present in the GMOs. The concept of known and unknown is usually related to the accepted or non accepted GMO in the food, in the grain or in the animal feed. Accepted GMO are well known and the presence of genetic elements in their genetic map is known. Unaccepted GMO are a priori unknown. The presence invention allows detecting the presence of an unknown GMO with or without prediction of its identity.

After hybridisation on the array, the target nucleotide sequence(s) can be detected by current techniques. Without labelling, preferred methods are the identification of the target by mass spectrometry now adapted to the arrays (US-A-5,821,060) or by intercalating agents followed by fluorescent detection (WO97/27329 or Fodor et al., Nature 364, p. 555 (1993)).

The labelled associated detections are numerous. A review of the different labelling molecules is given in W0 97/27317. They are obtained using either already labelled primer or by incorporation of labelled nucleotides during the copy or amplification step. A labelling can also be obtained by ligating a detectable moiety onto the RNA or DNA to be tested (a labelled oligonucleotide, which is ligated, at the end of the sequence by a ligase). Fragments of RNA or DNA can also incorporate labelled nucleotides at their 5'OH or 3'OH ends using a kinase, a transferase or a similar enzyme.

The most frequently used and preferred labels are fluorochromes like Cy3, Cy5 and Cy7 suitable for analysing an array by using commercially available array scanners (General Scanning, Genetic Microsystem,...).

Radioactive labelling, cold labelling or indirect labelling with small molecules recognised thereafter by specific ligands (streptavidin or antibodies) are common methods. The resulting signal of target fixation on the array is either fluorescent, colorimetric, diffusion, electroluminescent, bio- or chemiluminescent, magnetic, electric like impedometric or voltametric (US-A-5,312,527). A preferred method is based upon the use of the gold labelling of the bound target in order to obtain a precipitate or silver staining which is then easily detected and quantified by a scanner.

Quantification has to take into account not only the hybridisation yield and detection scale on the array (which is identical for target nucleotide sequence(s) and reference sequence(s)) but also the extraction, the amplification (or copying) and the labelling steps.

The method according to the invention may also comprise means for obtaining a quantification of target nucleotide sequence(s) by using a standard nucleotide sequence (external or internal standard) added at known concentration. A capture nucleotide sequence is also present on the array so as to fix the standard in the same conditions as the target nucleotide sequence (possibly after amplification or copying); the method comprising the step of quantification of a signal resulting from the formation of a double stranded nucleotide sequence formed by complementary base pairing between the capture nucleotide sequence(s) and the standard and the step of a correlation analysis of signal resulting from the formation of a double stranded nucleotide sequence with the signal resulting from the double stranded nucleotide sequence formed by complementary base pairing between capture nucleotide sequence(s) and the target in order to quantify the presence of the nucleotide sequence element to be detected and/or quantified in the biological sample.

Advantageously the standard is added in the biological sample or after the extraction step and is amplified or copied with the same primers and/or has a length and a GC content identical or differing from no more than 20% to the target nucleotide sequence. More preferably, the standard is designed as a competitive internal standard having the characteristics of the internal standard found in the document WO98/11253. This internal standard has a part of its sequence common to the target and a specific part which is different. It also has at or near its two ends sequence(s) which are complementary of the two primers used for amplification or copy of the target nucleotide sequence and similar GC content (WO98/11253).

In the preferred embodiment of this invention, the common part of the standard and the target nucleotide sequence, means a nucleotide sequence which is homologous to all target nucleotide sequences amplified by the same primers (i.e. which belong to the same family or organisms to be quantified).

Preferably, the hybridisation yield of the standard through this specific sequence is identical or differ no more than 20% from the hybridisation yield of the target sequence and quantification is obtained as described in WO 98/11253.

This standard nucleotide sequence, external and/or internal standard, is also advantageously included in a kit (device) or apparatus, possibly with all the media and means necessary for performing the different steps according to the invention (hybridisation and culture media, polymerase and other enzymes, standard sequence(s), labelling molecule(s), etc.).

Advantageously, the biochips also contain spots for the specific detection of some of the specifically amplified target nucleotide sequence(s) being amplified by specific primers pairs. Such spots are preferably added for confirmation of the identification of one organism or for discrimination between two organisms being very homologous or identical nucleotide sequence elements.

Advantageously, the biochips also contain spots with various concentrations (i.e. 4) of labelled capture nucleotide sequence(s). These labelled capture nucleotide sequences are spotted from known concentrations solutions and their signals allow the conversion of the results of hybridisation into absolute amounts. They also allow testing for the reproducibility of the detection.

The solid support (biochip) can be inserted in a support connected to another chamber and automatic machine through the control of liquid solution based upon the use of microfluidic technology. By being inserted into such a microlaboratory system, it can be incubated, heated, washed and labelled by automates, even for previous steps (like extraction of DNA, amplification by PCR) or the following step (labelling and detection). All these steps can be performed upon the same solid support.

Advantageously, the biochips also contain capture nucleotide sequences for the detection and/or identification and/or quantification of the plant species. in a particular embodiment, the identification of the plant species is also used for the identification of a particular GMOs specific of a plant species.

In a particular embodiment, a specific nucleotide sequence element of a plant, or organism, is used for the identification of plant or organism and in a special embodiment for the GMO's.

The quantification of the organism is preferably performed by quantification of the signal for a genetic element present in the organism.

In another embodiment the quantification of one organism is performed relative to its family by comparing the amount of a target nucleotide sequence specific of the organism to a target nucleotide sequence specific of the family. More specifically a quantification of a GMO is performed by comparison of the amount of a specific GMO element present in the sample compared to a plant marker. The ratio between the two allows calculating the percentage of GMO of one plant species in the sample.

Advantageously, the quantification of a specific organism is performed in term of copy number in the sample by comparing the amount of a target specific of the organism to a given number of standard copies added to the analyzed solution. In a preferred embodiment, the quantification of the nucleotide sequence element specific of a genetically modified plant is compared to the quantification of a standard sequence incorporated into the assay method at a known copy number. In another embodiment, the quantification of the nucleotide sequence element specific of a genetically modified plant is compared to the quantification of a gene or sequence of the non transformed part of the plant.

In a particular embodiment the quantification is performed after of the target nucleotide sequence and the standards by PCR using tailed primers and then using second primers identical or complementary to the tail(s). The first tailed primers, having a common tail, are directed against the specific elements and the standards or the plant marker as described by Knut et al. Nucleic Acids Res. 2003 Jun 1;31:e62.) Amplification is first performed for 5 to 20 cycles with the tailed primers and than for 5 to 40 cycles after destruction of the tailed primer and addition of the primer(s).

In a preferred embodiment the amplification is performed with tailed primers and the second tail primer(s) being both present from the beginning of the amplification without any destruction of the first primers given the use of an appropriate ratio between the tailed primers and the common tail primers, being at least 5 times lower and preferably 10 times lower.

Advantageously the quantification and the detection method for GMO are performed in the range of 0.1 to 5% presence of GMO in the sample compared to non GMO plant. Typical standard curve of standardization comprises GMO sample ranging from 0.1, 05, 1, 2 and 5% GMO in non GMO species. Regulation for labelling and or detection of known GMO containing sample is in the range of 0.9 and 0.5%. Unknown GMO are GMO which are not recognized as accepted for the commercialization and can not be found in the sample. Detection methods are required to detect as low as 0.1 % of such unknown GMO in the samples.

The present invention will be described in details in the following non-limiting examples in reference to the enclosed figures.

### Brief description of the drawings

Figure 1 represents the design of an array for the detection of the different elements of the GMOs and for their screening and identification as provided in example 3.

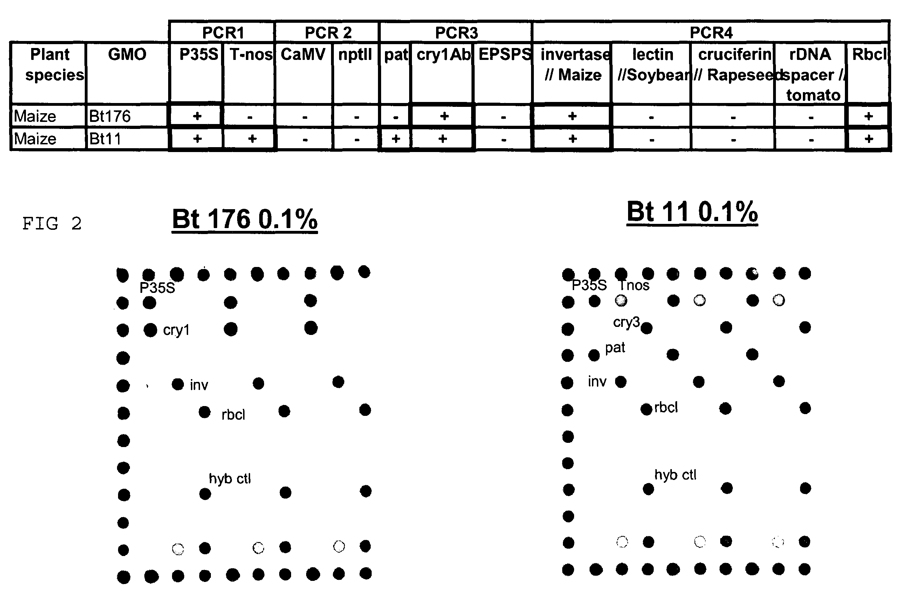

Figure 2 gives an example of result on the analysis of 2 GMO according to an array provide in the invention.

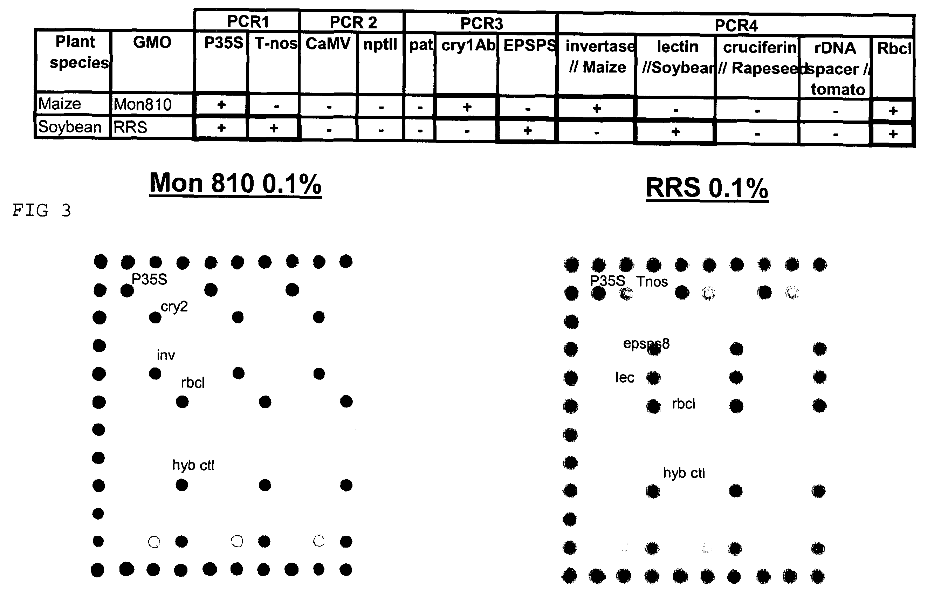

Figure 3 gives an example of result on the analysis of 2 GMO according to an array provide in the invention.

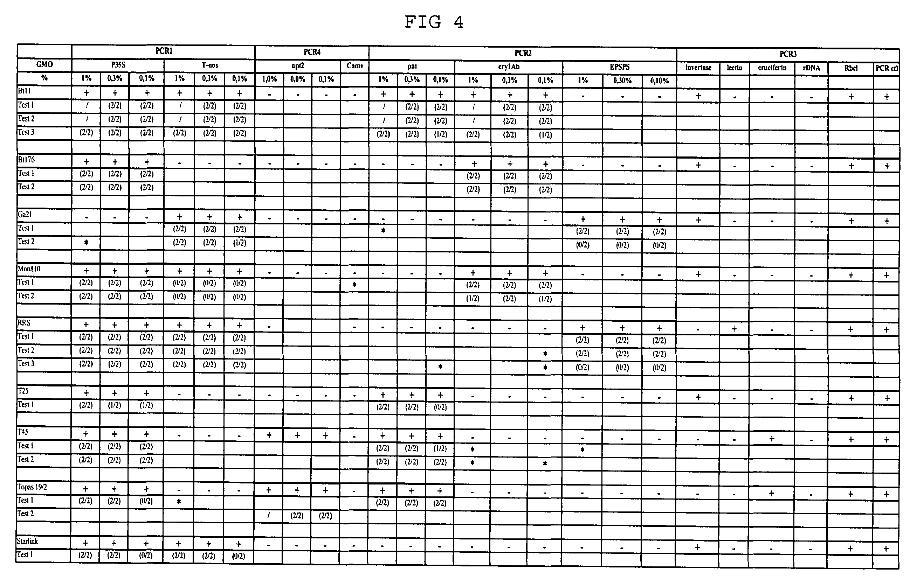

Figure 4 presents the overal results of the analysis of several GMO at various concentrations according to an array provide in the invention.

### Example 1: Detection of target sequence(s) on array

### Production of the capture nucleotide sequence(s) and of the targets

The target nucleotide sequences are obtained by PCR amplification using the following protocol.

The PCR are performed in a final volume of 25 µl containing: 1X Buffer Biotools including 2 mM MgCl₂, , 0.2 µM of each primer with one of the primer being biotinylated, 200 µM of each dATP, dCP, dGTP and 400 µM of dUTP,,, 1.25 U of Taq DNA polymerase Biotools, 0.5U of UNG. Samples are first incubated at 22°C for 10 minutes and then denatured at 94 °c for 5 min. Then 35 cycles of amplification are performed consisting of 30 sec at 94 °C, 40 sec at 56 °C and 1 min at 72 °C and a final extension step of 10 min at 72 °C. The incubation with UNG is for 10min at 20°c and the inactivation for 10min at 95°C. Water controls are used as negative controls of the amplification. The sequence(s) of the capture nucleotide sequence(s) are single stranded nucleotides.

The biochips also contains positive controls which are biotinylated polynucleotides of C2b2 gene hybridised on their corresponding capture nucleotide sequence and negative controls which are capture non specific nucleotide sequence(s).

### Capture nucleotide sequence immobilisation

The protocol described by Schena et al (Proc. Natl Acad. Sci. USA 93, 10614 (1996)) was followed for the grafting of aminated DNA to aldehyde derivatised glass. The aminated capture nucleotide sequence(s) were spotted from solutions at concentrations ranging from 150 to 3000 nM. The capture nucleotide sequence(s) were printed onto the silylated microscopic slides with a home made robotic device (250 µm pins from Genetix (UK) and silylated (aldehyde) microscope slides from Cell associates (Houston, USA)). The spots have 400 µm in diameter and the volume dispensed is about 0,5 nl. Slides were dried at room temperature and stored at 4 °C until used.

### Hybridisation

The array are covered with hybrizaton frames and are treated as proposed by the manufacturer (Eppendorf AG, Hamburg, Germany). At 50 µl of hybridisation solution (Genomic HybriBuffer, Eppendorf, AG) were added 45 µl of solution containing the amplicons, the blocking Hybridization solution, the positive hybridization controls and some water. The volume of the amplified target nucleotide sequence(s) was adjusted according to the level of detection required and the number of amplicon solutions to be incorporated. Typical experiment was performed with 4 amplicon solutions of 9µl each. The final solution was loaded on the array framed by a hybridisation chamber. For positive controls we added 2 nM biotinylated C2b2 probe of 27 bp to the solution; their corresponding capture nucleotide sequence(s) were spotted on the array. The array was pre-denatured by incubation with 0.5N NaOH beforehand. The hybridisation was carried out at 60° for 1 h. Slides were washed 4 times with a washing buffer.

### Colorimetric detection

The glass samples were incubated 45 min at room temperature with 800 µl of streptavidin labelled with colloidal gold 1000 x diluted in blocking buffer. After 5 washes with washing buffer, the presence of gold served for catalysis of silver reduction using a staining revelation solution (Silverquant reagent, Eppendorf, Hamburg, Germany). The slides were incubated 1 time 5 min with revelation mixture of Silverquant A and B solutions, then rinsed with water, dried and analysed using a microarray reader. Each array (slide) was then quantified by a specific quantification software.

### Fluorescence detection

The glass samples were incubated 45 min at room temperature with Cyanin 3 or Cyanin 5 labelled streptavidin. After washing the slides were dried before being stored at room temperature. The detection was performed in the array-scanner GSM 418 (Genetic Microsystem, Woburn, MA, USA). Each array (slide) was then quantified by a specific quantification software.

### Example 2: Detection of GMOs target sequence(s) on array

### Analysis of genetic elements of GMOs

The experiment is performed as proposed in example 1. Three PCR for the amplification of the following elements are performed :
PCR1:
   for Nos terminator : TTGAATCCTGTTGCCGGTCTT and
      CGCTATATTTTGTTTTCTATCGCG
   35S Promotor : CGTCTTCAAAGCAAGTGGATTG and
      TCTTGCGAAGGATAGTGGGATT
   nptll : CTCGACGTTGTCACTGAAG and GATGGATACTTTCTCGGCAG
   PCR control : CCACCTGCTGACCCCGTC and GGGACCCTCGCCCAGAAAC
PCR2:
   CaMV GTTGTTCTATTAGTTGCTCTT and ATGGCTAATCTTAATCAGATCC
   Pnos-nptII CCTCGGTATCCAATTAGAGTC and
      TTGTCTGTTGTGCCCAGTCAT
PCR3:
   Pat : GAGGCGCAAGGTTTTAAGTCT and CATCATGCCATCCACCATGC
   Cry1Ab : CMCWCAGAACAACAAYGTGC and GWGCWCKGATGATGCTCACG
   EPSPS : CAAGTCGMTYTCCMACCGG and CCTTGCCCGTATTGATGACG and
      GTCAAGGACCGCATTGCGA
The following specific sequence(s) are present on the capture nucleotide sequence(s)s for the different elements to be detected :
P35S: GTCATCCCTTACGTCAGTGGAGATAT
Tnos: GAGATGGGTTTTTATGATTAGAGTCC
nptIIA: GGGACTGGCTGCTATTGGGCGAA
gut (PCR control): GGGACTGGCTGCTATTGGGCGAA
pat1: CTGTGTATCCCAAAGCCTCATGCAA
cry1: CAGACGGTGGCTGAAGCCCTGTCG
cry2: GAGCCTGTGGGAAAAACCCTGCCT
cry3: CAACCTGTGGGAGAATCCTTGCCT
epsps7: CTCCTACTCGCCGCCCTGTCCGA
epsps8: TTCATGTTCGGCGGTCTCGCGAG
nptIIh: CCGCTTGGGTGGAGAGGCTATTC
CaMV: CGHTTTCATGGATTTTTGGTCACTG

The capture nucleotide sequence cry1 is specific for BT176, the cry2 for Mon 8110 and the cry3 for BT11. The capture nucleotide sequence EPSPS7 is specific for GA1 and the EPSPS2 for RRS.

The biochips also include the detection of the plant species. The amplification is performed in a separated tube.

The consensus primers for the amplification of the Rubisco activase and for the sucrose synthase are

| | **Primers** | | **Sequence(s)** |
|---|---|---|---|
| rubsico activase gene | ***Pra1*** | forward | 5'-ACAACCAGATGGTBAACGC-3' |
| | ***Pra2*** | reverse | 5'-GCCCAGTAYAARTTCTCCA-3' |
| | | | |
| sucrose synthase gene | ***Pss1*** | forward | 5'-GGTTTGGAGARRGGNTGGGG-3' |
| | ***Pss2*** | reverse | 5'-TCCAADATGTAVACAACCTG-3' |

The capture nucleotide sequence for the detection of the plant species are the following:
**Capture probes for the specific identification of six plant species:**

| **Capture probes names** | **Sequence (5' - 3')** | **Plant recognized** | **region recognized** |
|---|---|---|---|
| **TPss8** | **AGAGGAGGTGGATAGTCTCCTGTG** | **Maize** | **sucrose synthase gene** |
| **TPss9** | **AGAGAAGTTGAATTGACTCAAGGA** | **Soybean** | **sucrose synthase gene** |
| **TPss7** | **GAAGCAAGTGGATGGTGTCAAGCA** | **Rice** | **sucrose synthase gene** |
| **OTRcol2** | **TGATGGGAACACGTGCGTTTTCTT** | **Rapeseed** | **rubisco activase gene** |
| **PTRtom4** | **GTACCCTGGCGTTCTCTTGCTTGT** | **Tomato** | **rubisco activase gene** |
| **PTRbett2** | **TGATGGGTACACGAGCATTGTCCT** | **Sugar Beet** | **rubisco activase gene** |

### Example 3: Detection of GMOs target sequence(s) on array including specific plant identification

The detection of the GMO are performed with 3 PCR and capture probes as described in example 2. In the following example, a four PCR is added for the analysis of the plant species
- PCR4:
- Lectin (Soybean): CATTACCTATGATGCCTCCACC and
   AAGCACGTCATGCGATTCC
- Cruciferin (Rapeseed) : AGAGACGAAGGAAGCGAAGG
   and TGACCCATCTAATGCTGACG
- Invertase (Maize) : GCGCTCTGTACAAGCGTGC and
   GCAAAGTGTTGTGCTTGGACC
- rDNA (Tomato): GTTTCAAAAGTAACACGGCAA and
   GGCTTGATAAATGAACTCAACT
- Rbcl (Plant universal) :
   AGYCTTGATCGTTACAAAGG and
   AGGTCTAADGGRTAAGCTAC
the following specific sequence(s) are present on the capture probes
for the soybeam lectin: CTGCCACGGGACTCGACATACCT
for the rapeseed cruciferin: TTCAGAGTGCTGATGTAACCGAGCT
for the maize invertase: TTAGACGGGAAAACGAGAGGAAGC
for the tomato rDNA: CTCGCAATGGGGCTCAGAACGCA
for the universal plant:
ATAAGCAATATATTGATTTTCTTCTCCAGCAACGGGCTCGATGTGGTAGCATCGC

**Table I : Theoretical analysis of the genetic map for different GMO for the presence and absence of specific elements.**

| | | **PCR1** | | **PCR 2** | | **PCR3** | | | **PCR4** | | | | |
|---|---|---|---|---|---|---|---|---|---|---|---|---|---|
| **Plant species** | **GMO** | **P35S** | **T-nos** | **CaMV** | **nptII** | **pat** | **cry1Ab** | **EPSPS** | **invertase //Maize** | **lectin// Soybean** | **cruciferin// Rapeseed** | **rDNA spacer// tomato** | **Rbcl** |
| Maize | Bt11 | + | + | - | - | + | + | - | + | - | - | - | + |
| Maize | Bt176 | + | - | - | - | - | + | - | + | - | - | - | + |
| Maize | Ga21 | - | + | - | - | - | - | + | + | - | - | - | + |
| Maize | Mon810 | + | - | - | - | - | + | - | + | - | - | - | + |
| Soybean | RRS | + | + | - | - | - | - | + | - | + | - | - | + |
| Maize | T25 | + | - | - | - | + | - | - | + | - | - | - | + |
| Rapeseed | T45 | + | - | - | - | + | - | - | - | - | + | - | + |
| Rapeseed | Topas 19/2 | + | - | - | + | + | - | - | - | - | + | - | + |
| Maize | Starlink | + | + | - | - | - | - | - | + | - | - | - | + |

**Table II : Theoretical analysis of the genetic map for different GMO for the presence and absence of specific elements.**

| | PCR1 | | | PCR 2 | | PCR3 | | | | | | PCR4 | | | | |
|---|---|---|---|---|---|---|---|---|---|---|---|---|---|---|---|---|
| GMO | P35S | T-nos | nptll | CaMV | Pnos-nptll | pat | cry1Ab-1 | cry1Ab-2 | cry1Ab-3 | EPSPS-7 | EPSPS-8 | invertase | lectin | cruciferin | rDNA spacer II tomato | Rbcl |
| Bt11 | + | + | - | - | - | + | - | - | + | - | - | + | - | - | - | + |
| 8t176 | + | - | - | - | - | - | + | - | - | - | - | + | - | - | - | + |
| T25 | + | - | - | - | - | + | - | - | - | - | - | + | - | - | - | + |
| Mon810 | + | - | - | - | - | - | - | + | - | - | - | + | - | - | - | + |
| Mon 809 | + | + | + | - | - | - | - | + | - | - | + | + | - | - | - | + |
| NK603 | + | + | - | - | - | - | - | - | - | - | + | + | - | - | - | + |
| RRS//GTS 40/3/2 | + | + | - | - | - | - | - | - | - | - | + | - | + | - | - | + |
| Topas 19/2 | + | - | + | - | + | + | - | - | - | - | - | - | - | + | - | + |
| GT73 | - | - | - | - | - | - | - | - | - | - | + | - | - | + | - | + |
| Liberator L62 | + | - | - | - | - | + | - | - | - | - | - | - | - | + | - | + |
| Falcon GS 40/90 | + | - | - | - | - | + | - | - | - | - | - | - | - | + | - | + |
| MS1-RF1 | - | + | + | - | + | - | - | - | - | - | - | - | - | + | - | + |
| MS1-RF2 | - | + | + | - | + | - | - | - | - | - | - | - | - | + | - | + |
| MS8-RF3 | - | + | - | - | - | - | - | - | - | - | - | - | - | + | - | + |
| 1445 | - | + | + | - | - | - | - | - | - | - | + | - | - | - | - | + |
| 531 | - | - | + | - | - | - | - | - | - | - | - | - | - | - | - | + |
| Mon 863 | + | + | + | - | - | - | - | - | - | - | - | + | - | - | - | + |
| Mon810 x Mon 863 | + | + | + | - | - | - | - | + | - | - | - | + | - | - | - | + |
| TC 1507 | + | - | - | - | - | + | - | - | - | - | - | + | - | - | - | + |
| Maisgard/RR | + | + | - | - | - | - | - | + | - | - | + | + | - | - | - | + |
| Ga21 | - | + | - | - | - | - | - | - | - | + | - | + | - | - | - | + |

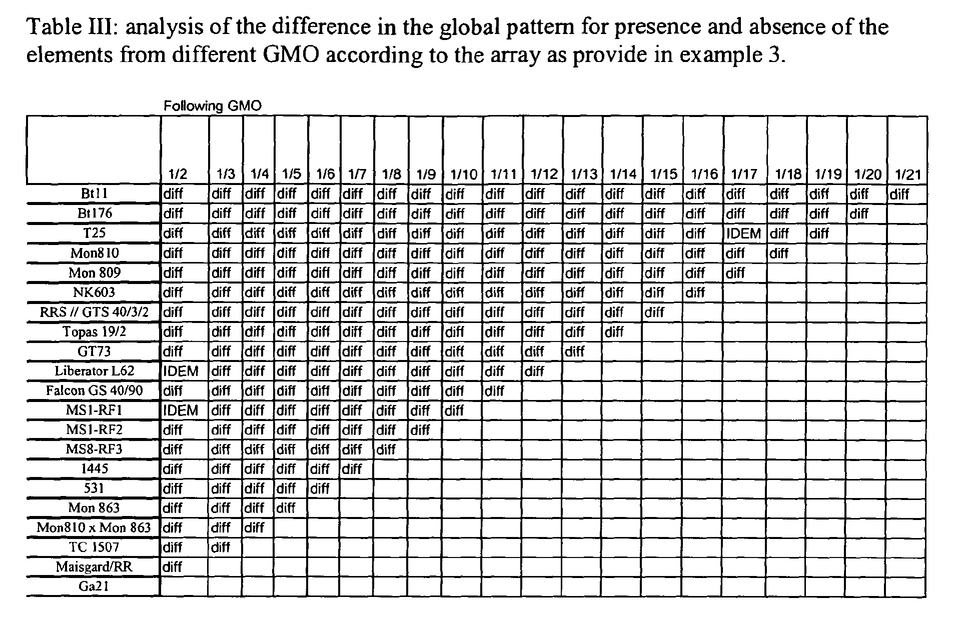

**Table IV : Analysis of several GMO according to an array provide in the invention. Intensities mean (S-B) for specific capture probes in function of the percentage of GMO tested.**

| | | ***Experimental value (grey intensities S-B)*** | | |
|---|---|---|---|---|
| ***GMO*** | ***Genetic element considered*** | *1,0%* | *0,3%* | *0,1%* |
| **Bt11** | P35S | 47382 | 26342 | 37526 |
| | Tnos | 33200 | 11966 | 26746 |
| | Pat | 41488 | 22619 | 27818 |
| | Cry1Ab3 | 48528 | 21553 | 18202 |
| | background | 3 | 3 | 3 |
| **Bt176** | P35S | 36592 | 43138 | 38691 |
| | Cry1Ab1 | 16390 | 14998 | 17813 |
| | background | 3 | 3 | 3 |
| **GA21** | Tnos | / | 45604 | 39585 |
| | EPSPS7 | / | 34575 | 10254 |
| | background | 3 | 3 | 3 |
| **Mon810** | P35S | 49102 | 47247 | 42652 |
| | Cry1Ab2 | 33470 | 43860 | 39035 |
| | background | 3 | 3 | 3 |
| **RRS//GTS 40/3/2** | P35S | 44923 | 32985 | 37356 |
| | Tnos | 41564 | 32428 | 28373 |
| | EPSPS8 | 52494 | 38037 | 29841 |
| | background | 3 | 3 | 3 |
| **T25** | P35S | / | 31574 | 18277 |
| | Pat | / | 18045 | 13163 |
| | background | 3 | 3 | 3 |
| **T45** | P35S | / | 36304 | 43618 |
| | Pat | / | 40325 | 42239 |
| | background | 3 | 3 | 3 |
| **Topas 19/2** | P35S | / | 35542 | 37253 |
| | nptll | / | / | 43176 |
| | Pat | / | 45301 | 39720 |
| | background | 3 | 3 | 3 |

## Claims

1. An identification and/or quantification method of different and multiple nucleotide sequence elements specific of a genetically modified plant in a biological sample, which comprises the steps of :
a. possibly extracting the nucleotide sequence elements from the biological sample;
b. amplifying or copying a nucleotide sequence element into target nucleotide sequences, using primer pairs which are able to amplify the said nucleotide sequence element being homologous with nucleotide sequence elements present in at least two different genetically modified plants;
c. possibly, labelling the obtained target nucleotide sequences;
d. putting into contact the obtained target nucleotide sequences with single stranded capture nucleotide sequences bound by a covalent link to an insoluble solid support at a specific location of a solid support surface;
e. detecting the binding of said target nucleotide sequences, by detecting a signal resulting from hybridization by complementary base pairing of the said target nucleotide sequence and its corresponding capture nucleotide sequence at the specific location, wherein the capture nucleotide sequence(s) are bound to the insoluble solid support at a specific location according to an array, having a density of at least 4 different bound single stranded capture nucleotide sequence(s)/cm² of solid support surface, wherein the capture nucleotide sequence comprises a sequence having between 10 and 200 bases, which allows a specific hybridization with the target nucleotide sequence to be detected and/or quantified;
f. repeating the steps b) to e) for a second, third, fourth or more different nucleotide sequence elements specific of the said genetically modified plant, and
g. identifying the genetically modified plant by the presence or absence of these different and multiple nucleotide sequence(s) elements, in the biological sample.

2. The method of claim 1, wherein the identification and/or the quantification of the multiple and different nucleotide sequence(s) elements are obtained by a comparison of the presence or absence of these nucleotide sequence(s) elements in the genome of a genetically modified plant.

3. The method according to the claims 1 or 2, wherein the nucleotide sequence(s) elements have a length comprised between about 100 and about 800 nucleotides, more preferably between about 100 and about 200 nucleotides, and correspond to at least a portion of exogenous nucleotide sequence(s) integrated into the genome of the genetically modified plant.

4. The method according to claim 1 or 2, wherein the capture nucleotide sequence able to hybridise with its corresponding target nucleotide sequence is separated from the surface of the solid support by a spacer of at least 6.8 nm.

5. The method according to claim 1 or 2, wherein the capture nucleotide sequence comprises a sequence having between 10 and 49 bases, which allows a specific hybridization with the target nucleotide sequence to be detected and/or quantified.

6. The method according to claim 3, wherein the exogenous nucleotide sequence(s) integrated into the genome of the plant are selected from the group consisting of antibiotic resistant genes, regulatory sequence(s), repeated sequences and/or genes coding for specific enzymatic activities.

7. The method according to any of the preceding claims, wherein the genetically modified plants are selected from the group consisting of the following varieties : BT11, BT176, Ga21, Mon 810, RRS, T25, T45, Topas19/2, Starlink,NK603,GT73, Liberator L62, Falcon GS 40/90, MS1-RF1, MS1-RF2, MS8-RF3, 1445, 531, Mon 863, Mon810xMON863, TC1507, Maisgard/RR.

8. The method according to any of the preceding claims, wherein the exogenous nucleotide sequence(s) are selected from the group consisting of the following promoters, terminators and/or markers sequence(s) : P35s, T-nos, nptII, pat, Cry1Ab and EPSPS.

9. The method according to any of the preceding claims, wherein the nucleotide sequence(s) elements are amplified into target nucleotide sequence(s) by using consensus primers.

10. The method according to any of the preceding claims, wherein the nucleotide sequence(s) elements are mRNA sequence(s) which are retro-transcribed into cDNA, with the same primer pair.

11. The method according to any of the preceding claims, wherein the target nucleotide sequence(s) corresponding to nucleotide sequence(s) elements specific of different genetically modified plants, are detected on the same capture nucleotide sequence(s).

12. The method according to any of the preceding claims, wherein the target nucleotide sequence(s) corresponding to nucleotide sequence(s) elements specific of different genetically modified plants, are detected on different capture nucleotide sequence (s).

13. The method according to any of the preceding claims, wherein the step b) of amplifying and/or copying the different nucleotide sequence(s) elements, specific of different genetically modified plants, are performed at the same time.

14. The method according to any of the preceding claims, wherein the different steps c) to e) of detecting and/or quantifying the target nucleotide sequence(s) corresponding to nucleotide sequence(s) elements specific of different genetically modified plants, are performed at the same time.

15. The identification method according to any of the preceding claims, wherein the spacer is a nucleotide sequence comprised between about 20 and about 150 bases.

16. The method according to any of the preceding claims, wherein the density of the capture nucleotides sequence(s) bound to the surface of the solid support, at specific location, is superior to 10 fmoles, preferably higher to 100 fmoles per cm² of the solid support surface.

17. The quantification method according to any of the preceding claims, wherein the amplification of nucleotide sequence(s) elements into target nucleotide sequence(s) are performed by PCR amplification, by first tailed primers and second primers identical or complementary to the tail(s).

18. The quantification method according to the claim 17, wherein the tailed primers are first destroyed before the amplification with the second tail primer(s).

19. The quantification method according to the claim 17 or 18, wherein the first tailed primers and the second tail primer(s) are both present from the first amplification step, with the tailed primers being at least in a concentration 5 times lower than the tail primer(s).

20. The quantification method according to any of the preceding claims 17 to 19, which further comprises an amplification step of other nucleotide sequence(s) than nucleotide sequence(s) elements and with primers pairs different than the ones used in the amplification and/or copying step of the nucleotide sequence(s) elements.

21. The quantification method according to claim 20, wherein the other nucleotide sequences submitted to another amplification step are nucleotide sequences specific of plant species, plant genus, or plant family detection.

22. The quantification method according to any of the preceding claims 17 to 21, wherein the quantification of the nucleotide sequence element specific of a genetically modified plant is compared to the quantification of a gene or sequence of the non transformed part of the plant.

23. The quantification method according to any of the preceding claims 17 to 22, wherein the quantification of the nucleotide sequence element specific of a genetically modified plant is compared to the quantification of a standard sequence incorporated into the assay method at a known copy number.

24. The method according to any of the preceding claims, wherein the insoluble solid support is selected from the group consisting of glasses, electronic devices, silicon supports, plastic supports, compact discs, filters, gel layers, metallic supports or a mixture thereof.

25. The method according to any of the preceding claims, wherein the nucleotide sequence(s) elements are RNA sequence(s) submitted to a retrotranscription of the 3' or 5' end by using consensus primer and possibly a stopper sequence.

26. The method according to any of the preceding claims, wherein the solid support bears capture nucleotide sequence(s) for the binding with the homologous target nucleotide sequence together with a consensus sequence for a common detection of all homologous or identical target sequence(s) corresponding to the same nucleotide sequence(s) elements present in the different genetically modified plants.

27. The method according to any of the preceding claims, wherein the method also includes the amplification of at least one standard which signal is used for quantification of the amount of a different and multiple nucleotide sequence element in the sample.

28. The method according to any of the preceding claims, wherein the method also includes the amplification of at least one standard which signal is used for quantification of the number of copies of the amount of a different and multiple nucleotide sequence element in the sample.

29. The method according to any of the preceding claims, wherein the quantification of the amount of a specific amount of a different and multiple nucleotide sequence element in the sample from a same family or a same species, in the sample is performed by comparing an amount of a specific element of the plant to the amount of an element present in all members of the same family, or a same specie.

30. A diagnostic and/or quantification kit which comprises means and media for performing the method according to any one of the preceding claims, preferably an insoluble solid support upon which single stranded capture nucleotide sequences are bound, said single stranded capture nucleotide sequences containing sequences of between about 10 and about 200 bases specific for at least three of P35s, T-nos, nptII, pat, Cry1Ab and EPSPS nucleotide sequences to be detected and/or quantified and having a total length comprised between about 30 and about 600 bases, said single stranded capture nucleotide sequence(s) being disposed upon the surface of the solid support according to an array with a density of at least 4 single stranded capture nucleotide sequence(s)/cm² of the solid support surface.

31. The diagnostic kit according to claim 30, wherein the capture nucleotide sequence(s) contain a specific sequence as provided in example 2.

32. The diagnostic kit according to claim 30 including a means in the form of a computer program for the analysis of the experimental results obtained on the detection and/or identification of different genetic elements and the identification of a organism.

33. The diagnostic kit according to claim 30 including a data bank with the composition of the genetic elements of the possible detectable organisms.
